# EUROPÄISCHE PATENTSCHRIFT

(11) **EP 0 710 604 B1**
(45) Veröffentlichungstag und Bekanntmachung des Hinweises auf die Patenterteilung: **18.03.1998**
(21) Anmeldenummer: 95115506.8
(22) Anmeldetag: 30.09.1995
(51) Int. Cl.: B65B 3/02, B29C 49/48

(54) **Blasformverfahren zum Herstellen eines verschlossenen Behältnisses und nach diesem Verfahren hergestelltes Behältnis**
Blow moulding method for producing closed containers and containers produced by this method
Procédé de moulage par soufflage de récipients fermés et récipients réalisés selon ledit procédé

(30) Priorität: 03.11.1994 DE 4439231
(43) Veröffentlichungstag der Anmeldung: 08.05.1996
(73) Patentinhaber: Hansen, Bernd, Dipl.-Ing., D-74429 Sulzbach-Laufen (DE)
(72) Erfinder: Hansen, Bernd, D-74429 Sulzbach-Laufen (DE); Leu, Willy, CH-5057 Reitnau (CH)
(74) Vertreter: Patentanwälte Bartels und Partner

(56) Entgegenhaltungen:
- EP-A- 0 272 672
- EP-A- 0 425 264
- DE-A- 3 445 542
- DE-A- 3 635 402
- US-A- 4 655 355
- US-A- 5 054 267
- PATENT ABSTRACTS OF JAPAN vol. 008 no. 112 (M-298) ,25.Mai 1984 & JP-A-59 020629 (MITSUBISHI YUKA KK) 2.Februar 1984,
- PATENT ABSTRACTS OF JAPAN vol. 011 no. 188 (M-599) ,17.Juni 1987 & JP-A-62 016124 (MEIHOU KOGYO KK) 24.Januar 1987,

## Beschreibung

Die Erfindung betrifft ein Blasformverfahren zum sterilen Herstellen eines verschlossenen, mit einer Substanz gefüllten Behältnisses, das die Merkmale des Oberbegriffs des Anspruchs 1 aufweist, sowie ein nach diesem Verfahren hergestelltes Behältnis.

Durch die DE-A-36 35 402 ist ein verfahrbarer Formboden bekannt, mit dem sich mit einem Blasformverfahren ein Behältnis herstellen läßt, das ohne schrägen Übergang zwischen Kopf und Körper einen relativ großen Aufblasverhältniswert aufweist. Bei diesem bekannten Blasformverfahren mit bewegbarem Formboden kann nicht ausgeschlossen werden, daß nach dem Befüllen des Behältnisses und Schließen des Kopfes sich noch Luft in diesem befindet.

Ebenso wenig ist dies sichergestellt bei dem bekannten Verfahren nach der DE-A-34 45 542, die die Herstellung eines hermetisch dichten Behälters betrifft mit den Herstellschritten Formen, Füllen und Verschließen, wobei in den Kopf des Behältnisses ein Einlegeteil in Form eines zylindrischen Schraubteils einsetzbar ist.

Ausgehend von diesem Stand der Technik liegt der Erfindung die Aufgabe zugrunde, ein Blasformverfahren zu schaffen, mit dem Behältnisse ohne schrägen Übergang zwischen Kopf und Körper mit einem relativ großen, vorzugsweise über dem Wert 4 liegenden Aufblasverhältnis, also dem Verhältnis zwischen Körperdurchmesser und Kopfdurchmesser hergestellt werden können, das es außerdem ermöglicht, im Bereich des Übergangs eine größere Wandstärke als im Bereich der Mantelfläche des Körpers zu erreichen und das ferner das Einsetzen eines Einlegekörpers in den Kopf des Behältnisses sowie eine vollständige, also luftfreie Befüllung erlaubt. Diese Aufgabe löst ein Verfahren mit den Merkmalen des Anspruches 1.

Auch bei einem großen Aufblasverhältnis gewährleistet das Verschieben des Formbodens gegen den Kopf hin während des Aufweitens des sich in der Form befindenden Schlauchabschnittes die Erzielung eines neigungsfreien Übergangs zwischen Kopf und Körper des Behältnisses sowie einer Wandstärke im Bereich des Übergangs zwischen Kopf und Körper, die sogar wesentlich größer sein kann als die Wandstärke im Bereich der Mantelfläche des Körpers. Ferner wird durch die Bewegung des Formbodens gegen den Kopf hin nach dem Befüllen des Behältnisses sichergestellt, daß das Behältnis beim Schließen des Kopfes vollständig luftfrei ist oder allenfalls noch eine vernachlässigbare kleine Luftmenge im Inneren des Einlegekörpers enthält.

Der Formboden wird bei seinem ersten Bewegungsvorgang bis in die der Größe des Behälters im verschlossenen Zustand entsprechende Position bewegt und während des Füllvorganges ein Stück weit zurückbewegt. Das Behältnis enthält dann einen geringen Überschuß an Füllsubstanz, die durch eine Bewegung des Formbodens in die der Größe des Behältnisses im verschlossenen Zustand entsprechende Position verdrängt werden kann, wodurch sichergestellt ist, daß das Behältnis tatsächlich vollständig und damit auch luftfrei gefüllt ist.

Da der Kunststoff bei der Extrusion bereits einen Sterilisationsprozeß durchläuft, das Behältnis mit steriler Luft geblasen und mit einem sterilen Produkt gefüllt wird und der gegebenenfalls einzusetzende Einlegekörper aseptisch zugeführt wird, ist die vielfach verlangte Sterilität zuverlässig gewährleistet.

Selbstverständlich kann statt der Erzeugung eines Überdruckes im Inneren des Schlauches zu seiner Aufweitung auch oder zusätzlich ein auf seine Außenseite wirkender Unterdruck vorgesehen werden.

Bei einer bevorzugten Ausführungsform wird während des ersten Bewegungsvorganges des Formbodens die Blasluft zunächst nur mit geringem Druck eingeblasen. Erst kurz vor dem Erreichen der Endposition des Formbodens wird dann der Druck auf den vollen Wert erhöht, um gegen Ende des Formvorganges eine vollständige Anlage des Formlings an der Innenwand der Form sicherzustellen, ohne zuvor den Verformungsvorgang des Schlauches ungünstig zu beeinflussen.

Vorteilhafterweise werden der Überschuß an eingefüllter Substanz und die zunächst im Behältnis noch vorhandene Luft nicht allein durch das Bewegen des Formbodens gegen den Kopf hin entfernt. Vielmehr wird vorzugsweise nach dem Befüllen des Behältnisses und während der sich daran anschließenden Bewegung des Formbodens die Luft und gegebenenfalls die zuviel eingefüllte Substanz abgesaugt. Da zweckmäßigerweise der Einlegekörper schon zuvor in den Kopf eingelegt worden ist, erfolgt dieses Absaugen vorzugsweise durch den Einlegekörper hindurch. Sofern dieser nicht durchstochen werden kann oder durchstochen werden soll, ist in ihm ein Längskanal vorzusehen.

In der Regel reicht das Andrücken der Schlauchwandung an den Einlegekörper aus, um eine feste und dichte Verbindung herzustellen. Man kann aber auch, falls erforderlich, den Einlegekörper mit dem ihn umschließenden Kopf längs seines Umfanges verschweißen, beispielsweise mittels Ultraschall oder eines Laserstrahles.

Der Erfindung liegt auch die Aufgabe zugrunde, ein nach dem erfindungsgemäßen Verfahren hergestelltes Behältnis zu schaffen, das manuell ohne Schwierigkeiten für das Abgeben der eingefüllten Substanz in seiner Länge reduziert werden kann. Diese Aufgabe löst ein Behältnis mit den Merkmalen des Anspruches 8, weil dank der unterschiedlichen Wandstärken im Bereich des Übergangs und des Bodens einerseits sowie des Mantels des Körpers andererseits dem Übergang und dem Boden eine ausreichende Biegesteifigkeit und dem Mantel des Körpers eine gute Verformbarkeit gegeben werden kann. Es läßt sich deshalb ein Behältnis realisieren, bei dem der Boden in Anlage an den Übergang zwischen Kopf und Körper gebracht werden kann.

Zur Versteifung des Bodens und des Übergangs zwischen Kopf und Körper können von diesen beiden Teilen das eine wenigstens eine Vertiefung und das andere wenigstens eine auf diese Vertiefung ausgerichtete, vorspringende Materialpartie aufweisen.

Bei einer bevorzugten Ausführungsform ist der Einlegekörper in Querrichtung geteilt oder teilbar, wobei diese Teilungsstelle im Bereich einer Sollbruchstelle des Kopfes liegt, damit beim Öffnen des Behältnisses durch Abreißen eines Teils des Kopfes auch der Einlegekörper geteilt wird. Vorzugsweise sind deshalb in axialer Richtung formschlüssige Verbindungen zwischen dem Einlegekörper und dem Kopf vorgesehen, wodurch sichergestellt ist, daß beim Öffnen die beiden Teile des Einlegekörpers in Verbindung mit den sie umgebenden Teilen des Kopfes bleiben.

Der Einlegekörper kann beispielsweise eine hohle Nadel enthalten, die in demjenigen Teil des Einlegekörpers, der sich in dem unlösbar mit dem Körper verbundenen Teil des Kopfes befindet, festgelegt ist und in einen Längskanal des anderen Teils des Einlegekörpers ragt. Beim Öffnen des Behältnisses wird dann diese hohle Nadel auf der über den sie tragenden Teil des Einlegekörpers überstehenden Länge freigelegt. Durch diese hohle Nadel hindurch kann beim Herstellvorgang Luft und überschüssiges Füllgut abgesaugt werden.

Um das Aufbringen der für das Öffnen des Behältnisses erforderlichen Kraft zu erleichtern, ist vorzugsweise an demjenigen Teil des Kopfes, der das vom Körper wegweisende Ende des Einlegekörpers bedeckt, ein Griffteil angeformt, der vorteilhafterweise in Längsrichtung des Kopfes von diesem absteht.

Im folgenden ist die Erfindung anhand eines in der Zeichnung dargestellten Ausführungsbeispiels im einzelnen erläutert. Es zeigen je in vergrößertem Maßstab
- Fig. 1: einen Längsschnitt des Ausführungsbeispiels im fertigen Zustand,
- Fig. 2: einen Längsschnitt des Ausführungsbeispiels im geöffneten und auf das minimale Volumen reduzierten Zustand, wobei der abgerissene Teil des Kopfes in einer Position während des Öffnen des Behältnisses dargestellt ist,
- Fig. 3: einen unvollständig und schematisch dargestellten Längsschnitt der Blasform.

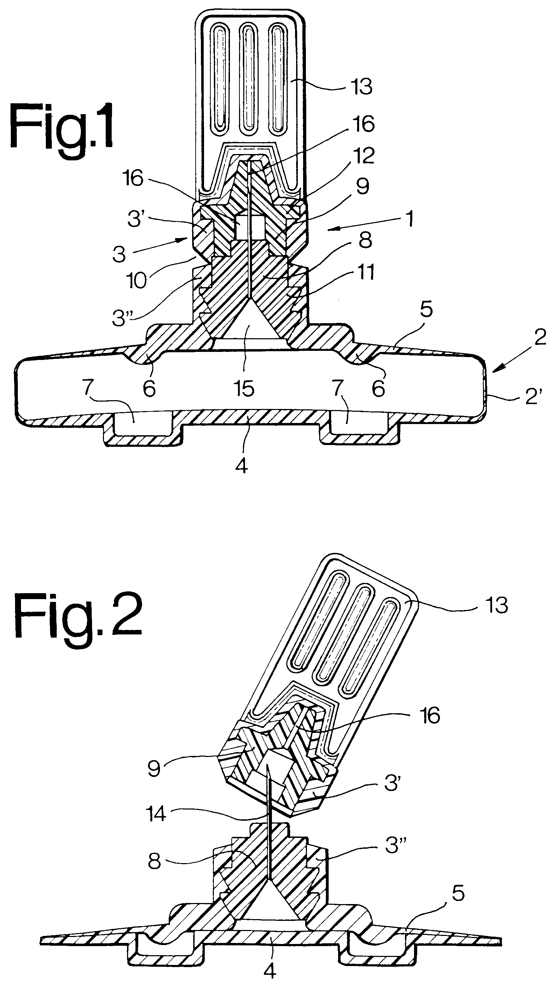

Ein als Ganzes mit 1 bezeichnetes Behältnis, das wegen seines geringen Volumens als Ampulle bezeichnet werden kann, besteht aus einem im wesentlichen zylindrischen Körper 2, dessen axiale Länge wesentlich geringer ist als sein Durchmesser, und einem Kopf 3, der sich an das dem Boden 4 gegenüberliegende, stirnseitige Ende des Körpers 2 gleichachsig anschließt. Der Außendurchmesser des Körpers 2 ist etwa viermal so groß wie der Außendurchmesser des Kopfes 3. Wie Fig. 1 zeigt, ist der Übergang 5 von der zylindrischen Wandfläche 2' zum Kopf 3 praktisch neigungsfrei, d.h., er liegt zumindest nahezu in einer Radialebene. Wie Fig. 1 ferner zeigt, weist die unmittelbar an den Kopf anschließende Ringzone des Übergangs 5 eine sehr große Wandstärke auf. Von dieser Ringzone aus verringert sich die Wandstärke des Übergangs 5 zur Wandfläche 2' hin kontinuierlich auf die Dicke der Wandfläche 2'. Man kann aber auch von der Verdickung der Ringzone absehen und die Wandstärke des Überganges 5 zur Wandfläche 2' vom Kopf 3 an radial nach außen stufenlos abnehmen lassen.

Am Übergang von der Ringzone zum radial nach außen hin anschließenden Bereich des Übergangs 5 ist innen ein zum Boden hin vorspringender, zum Kopf 3 konzentrischer Ringwulst 6 angeformt. Dieser ist auf eine zu ihm hin offene Ringnut 7 des Bodens 4 ausgerichtet, dessen Wanddicke innerhalb der Ringnut 7 konstant und mindestens halb so groß ist wie die Dicke der Ringzone des Übergangs 5. Die die Ringnut 7 begrenzende Materialpartie steht nach außen über den Boden 4 über. Außerhalb der Ringnut 7 verringert sich die Wandstärke des Bodens 4 zur Wandfläche 2' hin kontinuierlich auf deren Wert. Dank der relativ großen Wandstärke sowie der Versteifung durch den Ringwulst 6 und die Ringnut 7 sind der Übergang 5 und der Boden 4 so biegesteif, daß die für die Verformung der Wandfläche 2' erforderliche Kraft zu keiner nennenswerten Deformation von Übergang 5 und Boden 4 führt. Der Boden 4 kann deshalb, wie Fig.2 zeigt, in Anlage an den Übergang 5 gebracht werden. Dabei ragt der Ringwulst 6 in die Ringnut 7.

In den Kopf 3 ist ein Einlegekörper eingesetzt, der wie der Körper 2 und der Kopf 3 aus Kunststoff besteht. Vorzugsweise hat aber der Einlegekörper eine größere Härte als der den Körper 2 und den Kopf 3 bildende Kunststoff. Im Ausführungsbeispiel besteht der Einlegekörper aus einem ersten Teil 8, der bis in die dicke Ringzone des Übergangs 5 hineinragt, und aus einem sich gleichachsig an die dem Innenraum des Körpers 2 abgekehrten Stirnfläche des ersten Teiles 8 anschließenden zweiten Teil 9. Im Ausführungsbeispiel ist der zweite Teil 9 auf einen zapfenartigen Ansatz des ersten Teils 8 aufgesteckt. Der zweite Teil 9 könnte aber auch einstückig mit dem ersten Teil 8 ausgebildet sein und am Übergang zwischen beiden Teilen eine Sollbruchstelle aufweisen. Mit einer solchen Sollbruchstelle ist der Kopf 3 versehen, der hierzu etwa in Höhe des Übergangs vom ersten Teil 8 zum zweiten Teil 9 eine von außen her radial eindringende, umlaufende Nut 10 aufweist, welche die im übrigen relativ große Wandstärke des Kopfes 3 auf einen minimalen Wert reduziert. Die umlaufende Nut 10 hat ein dreieckförmiges Querschnittsprofil, um den abzubrechenden Teil des Kopfes 3 zum Zwecke des Trennens kippen zu können. Ein Flansch oder, wie dargestellt, ringförmige, ein Sägezahnprofil ergebende Zähne 11, die an der Außenmantelfläche des ersten Teils 8 des Einlegekörpers vorgesehen sind, stehen in Eingriff mit dem den Kopf 3 bildenden Material und ergeben eine in axialer Richtung formschlüssige Verbindung zwischen dem ersten Teil 8 und dem Kopf 3. Eine entsprechende Verbindung zwischen dem zweiten Teil 9 und dem Kopf 3 wird durch einen radial nach außen abstehenden Flanschteil 12 erreicht, den das Material des Kopfes 3 umgreift.

An das vom Körper 2 wegweisende Ende des Kopfes 3 ist an diesen ein in Kopflängsrichtung abstehender Griffteil 13 angeformt, mittels dessen zum Öffnen des Behältnisses 1 der obere Abschnitt 3' des Kopfes 3 relativ zum unteren Abschnitt 3'' so weit gekippt wird, bis ein Abriß an der im Grund der umlaufenden Nut 10 liegenden Sollbruchstelle erfolgt.

Im Ausführungsbeispiel ist in der Längsachse des ersten Teils 8 des Einlegekörpers eine Hohlnadel 14 festgelegt, auf deren dem Boden 4 zugewandtes Ende ein im Teil 8 vorgesehener Trichter 15 ausgerichtet ist. Der über das dem Boden 4 abgekehrte Ende des ersten Teils 8 des Einlegekörpers überstehende Abschnitt der Hohlnadel 14 liegt, wie Fig. 1 zeigt, vor dem Öffnen des Behältnisses in einem zentralen Durchgangskanal 16 des zweiten Teils 9 des Einlegekörpers. Dieser zentrale Durchgangskanal 16 hat im Anschluß an den ersten Teil 8 einen im Vergleich zum Durchmesser der Hohlnadel 14 wesentlich größeren Durchmesser im übrigen aber einen an den Durchmesser der Hohlnadel 14 angepaßten Wert. Das dem ersten Teil 8 abgekehrte Ende des zentralen Durchgangskanals 16 ist von dem den Einlegekörper umhüllenden Material des Kopfes 3 dicht verschlossen.

Das vorstehend beschriebene Behältnis 1 wird im Blasformverfahren aus einem extrudierten Schlauchabschnitt hergestellt. Außer den den Körper 2 formenden Hauptbacken 17 weist diese Form Kopfbacken 18 sowie einen Formboden 19 auf, der in axialer Richtung, also gegen die Kopfbacken 18 hin und von diesen weg, in den Hauptbacken 17 bewegt werden kann.

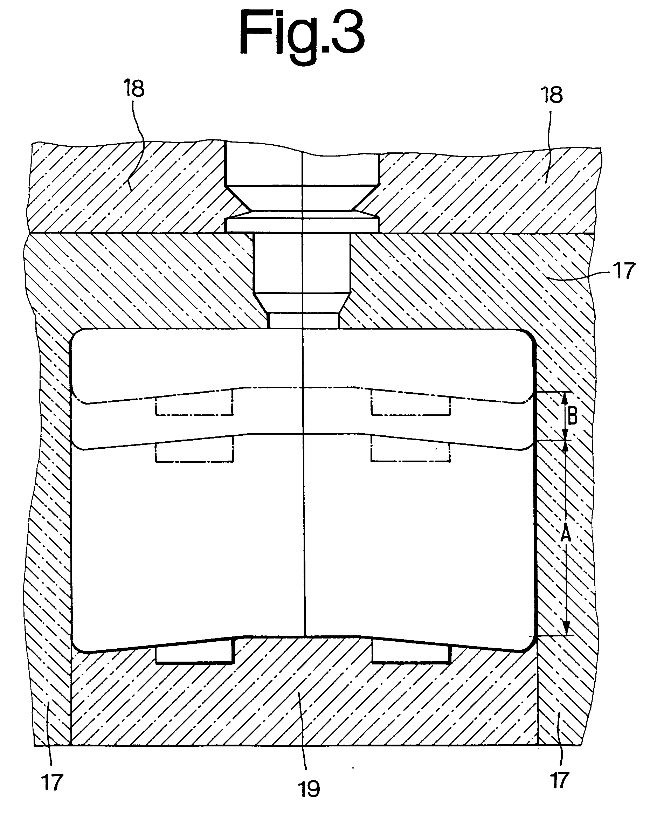

Nachdem der extrudierte Schlauch, der im Durchmesser etwas größer ist als der Durchmesser des Kopfes 3, in die Form eingebracht worden ist, wird er an seinem unteren Ende verschlossen. Der Formboden 19 befindet sich dabei in dem in Fig. 3 dargestellten, größtmöglichen Abstand von den Kopfbacken 18. In der nun geschlossenen Form beginnt jetzt der Blasvorgang. Hierzu wird in den später den Kopf 3 bildenden Abschnitt des Schlauches ein Blas- und Fülldorn eingeführt, aus dem zunächst Luft mit stark vermindertem Druck ausströmt. Gleichzeitig fährt der Formboden 19 von der Position größten Abstandes von den Kopfbacken 18 in die Position geringsten Abstandes. Er führt also den aus den Teilhüben A und B bestehenden Gesamthub aus. Kurz ehe der Formboden 19 die Endposition erreicht hat, wird der Druck der Luft auf den Normalwert erhöht. Wenn die Endposition erreicht ist, wird die Luftzufuhr abgeschaltet und der Füllvorgang begonnen. Dabei wird der Formboden 19 um die dem Teilhub B entsprechende Strecke zurückgezogen.

Nach beendeter Füllung und dem Entfernen des Blas- und Fülldornes wird mittels eines Greifers der aus den beiden Teilen 8 und 9 zusammengesetzte Einlegekörper in den zur Bildung des Kopfes 3 dienenden Abschnitt des Schlauches eingesetzt und dieser Abschnitt des Schlauches mittels der Kopfbacken an die Mantelfläche des Einlegeteils angepreßt. Ferner wird mit Hilfe des Greifers an die nach außen weisende Mündungsöffnung des zentralen Durchgangskanals 16 des Einlegeteils ein Vakuum angelegt, durch welches im Behältnis vorhandene Luft und überschüssiges Füllgut abgesaugt wird. Unterstützt wird das Entfernen der Luft und des überschüssigen Füllgutes durch eine Bewegung des Formbodens 19 um die Strecke des Hubes B gegen die Kopfbacken 18 hin in die Position geringsten Abstandes von den Kopfbacken 18. Danach hebt der Greifer ab und die Kopfbacken 18 verschließen den Kopf 3 hermetisch durch Verschweißen des Schlauchmaterials unter gleichzeitiger Bildung des Griffteiles 13.

Sofern erforderlich kann die den Kopf 3 bildende Wand mit dem Einlegekörper rundum verschweißt werden, beispielsweise mit Hilfe eines Laserstrahles oder mit Hilfe von Ultraschall.

Wenn das Füllgut dem Behältnis 1 entnommen werden soll, wird der obere Abschnitt 3' des Kopfes 3 vom unteren Abschnitt 3'' abgebrochen. Dabei löst sich der zweite Teil 9 des Einlegekörpers vom ersten Teil 8, wodurch die Hohlnadel 14 freigegeben wird. Wird nun der Boden 4 gegen den Übergang 3 gedrückt, tritt aus der Hohlnadel 14 das Medium tropfenweise oder kontinuierlich aus.

## Patentansprüche

1. Blasformverfahren zum Herstellen eines verschlossenen, mit einer Substanz gefüllten Behältnisses (1), an dessen Körper (2) sich ein Kopf (3) anschließt, von dem zum Öffnen des Behältnisses (1) ein Teil abtrennbar ist, wobei zur Erzielung eines Durchmessers des Körpers (2), der wesentlich größer ist als der Durchmesser des Kopfes (3), und eines Überganges vom Kopf (3) zum Körper (2), der zumindest nahezu in einer radialen Ebene liegt,
a) in eine einen axial verschiebbaren Formboden (19) aufweisende Blasform ein Abschnitt eines extrudierten Schlauches eingebracht wird, dessen Durchmesser zunächst allenfalls wenig größer ist als der Durchmesser des Kopfes (3),
b) nach dem Schließen des den Boden des Behältnisses (1) bildenden Schlauchendes in den Schlauch Luft eingeblasen und gleichzeitig der Formboden (19) gegen die den Kopf (3) formenden Kopfbacken (18) der Blasform hin bewegt wird,
c) der Kopf (3) geformt und unter Anlegen an die Mantelfläche eines gegebenenfalls vorhandenen Einlegekörpers (8,9) dicht verschlossen wird,
dadurch gekennzeichnet, daß
d) nach dem Befüllen des Behältnisses (1) und dem gegebenenfalls erfolgenden Einlegen des Einlegekörpers (8,9) in den der Bildung des Kopfes (3) dienenden Abschnitt des Schlauches der Formboden (19) noch weiter gegen die Kopfbacken (18) bewegt wird, bis das Behältnis (1) luftfrei ist und
e) der Formboden (19) bei seinem ersten Bewegungsvorgang bis in die der Größe des Behältnisses (1) im verschlossenen Zustand entsprechende Position bewegt und während des Füllvorganges ein Stück weit zurückbewegt wird.

2. Verfahren nach Anspruch 1, dadurch gekennzeichnet, daß der Formboden (19) nach dem Befüllen um den Weg gegen den Kopf (3) bewegt wird, um den er zuvor zurückbewegt worden ist.

3. Verfahren nach Anspruch 1 oder 2, dadurch gekennzeichnet, daß während des ersten Bewegungsvorgangs des Formbodens (19) die Luft zunächst mit vermindertem Druck eingeblasen wird und erst kurz vor dem Erreichen der Endposition der Druck auf den vollen Wert erhöht wird.

4. Verfahren nach einem der Ansprüche 1 bis 3, dadurch gekennzeichnet, daß nach dem Befüllen des Behältnisses (1) und während der sich dann anschließenden Bewegung des Formbodens (19) gegen den Kopf (3) hin Luft und gegebenenfalls zuviel eingefüllte Substanz abgesaugt werden.

5. Verfahren nach Anspruch 4, dadurch gekennzeichnet, daß das Absaugen von Luft und zuviel eingefüllter Substanz durch den gegebenenfalls eingelegten Einlegekörper (8,9) hindurch erfolgt.

6. Verfahren nach einem der Ansprüche 1 bis 5, dadurch gekennzeichnet, daß der gegebenenfalls vorhandene Einlegekörper (8,9) mit dem ihm umschließenden Kopf (3) längs seines Umfanges verschweißt wird.

7. Nach dem Verfahren gemäß einem der Ansprüche 1 bis 6 hergestelltes Behältnis (1), dadurch gekennzeichnet, daß die Dicke seiner Wand in dem die Mantelfläche (2') des Körpers (2) bildenden Bereich wesentlich geringer ist als in dem Übergangsbereich (5) vom Körper (2) zum Kopf (3).

8. Behältnis nach Anspruch 7, dadurch gekennzeichnet, daß von den beiden den Boden (4) und den Übergang (5) vom Körper (2) zum Kopf (3) bildenden Bereichen (5) der eine wenigstens eine Vertiefung (7) und der andere wenigstens eine auf diese Vertiefung ausgerichtete, vorspringende Materialpartie (6) aufweist.

9. Behältnis nach Anspruch 7 oder 8, dadurch gekennzeichnet, daß der Einlegekörper (8,9) in Querrichtung geteilt oder teilbar ist und diese Teilungsstelle im Bereich einer Sollbruchstelle des Kopfes (3).

10. Behältnis nach Anspruch 9, dadurch gekennzeichnet, daß zumindest der beim Öffnen des Behältnisses (1) in dessen Kopf (3) verbleibende Teil (8) des Einlegekörpers (8,9) in seiner Außenmantelfläche mit wenigstens einer Ringnut und/oder einem Ringwulst (11) für eine formschlüssige Verbindung in axialer Richtung zwischen dem Einlegekörper (8,9) und dem Kopf (3) versehen ist.

11. Behältnis nach einem der Ansprüche 7 bis 10, dadurch gekennzeichnet, daß an denjenigen Teil (3') des Kopfes (3), der das vom Körper (2) wegweisende Ende des Einlegekörpers (8,9) bedeckt, ein Griffteil (13) angeformt ist.

12. Behältnis nach Anspruch 11, dadurch gekennzeichnet, daß das Griffteil (13) in Längsrichtung des Kopfes (3) von diesem absteht.

## Claims

1. Blow moulding method for producing a closed container (1) filled with a substance, adjoining the body (2) of which is a head (3), from which a part can be separated for opening the container (1), in which case for achieving a diameter of the body (2), which is substantially larger than the diameter of the head (3), and a transition from the head (3) to the body (2), which lies at least approximately in a radial plane,
a) a section of an extruded tube is introduced into a blow mould comprising an axially displaceable mould base (19), the diameter of which tube is first of all in any case a little larger than the diameter of the head (3),
b) after closing the end of the tube forming the base of the container (1), air is blown into the tube and at the same time the mould base (19) is moved towards the head jaws (18) of the blow mould forming the head (3),
c) the head (3) is moulded and is sealed hermetically by application against the surface of an insertion body (8, 9) present if necessary,
characterised in that
d) after the filling of the container (1) and the insertion of the insertion body (8, 9) in the section of the tube serving to form the head (3), which possibly takes place, the mould base (19) is moved still further towards the head jaws (18), until the container (1) is free from air and
e) upon its first movement process, the mould base (19) is moved into the position corresponding to the size of the container (1) in the closed state and during the filling process is moved back a bit.

2. Method according to Claim 1, characterised in that after filling, the mould base (19) is moved towards the head (3) by the distance by which it had been moved back previously.

3. Method according to Claims 1 or 2, characterised in that during the first movement process of the mould base (19), the air is blown in first of all at reduced pressure and the pressure is increased to full value only shortly before reaching the end position.

4. Method according to one of Claims 1 to 3, characterised in that after filling the container (1) and during the subsequent movement of the mould base (19) towards the head (3), air and any substance which was possibly introduced in excess, are removed by suction.

5. Method according to Claim 4, characterised in that the removal by suction of air and any substance introduced in excess takes place through the possibly inserted insertion body (8, 9).

6. Method according to one of Claims 1 to 5, characterised in that the possibly present insertion body (8, 9) is welded along its periphery to the head (3) surrounding it.

7. Container (1) produced in accordance with the method according to one of Claims 1 to 6, characterised in that the thickness of its wall in the region forming the surface (2') of the body (2) is substantially less than in the transition region (5) from the body (2) to the head (3).

8. Container according to Claim 7, characterised in that of the two regions (5) forming the base (4) and the transition (5) from the body (2) to the head (3), one comprises at least one recess (7) and the other comprises at least one projecting material part (6) aligned with this recess.

9. Container according to Claim 7 or 8, characterised in that the insertion body (8, 9) is divided or can be divided in the transverse direction and this division point is in the region of a predetermined breaking point of the head (3).

10. Container according to Claim 9, characterised in that at least the part (8) of the insertion body (8, 9) remaining in its head (3) at the time of opening of the container (1) is provided in its outer surface with at least one annular groove and/or one annular bead (11) for a form-locking connection in the axial direction between the insertion body (8, 9) and the head (3).

11. Container according to one of Claims 7 to 10, characterized in that a handle part (13) is integrally formed on that part (3') of the head (3), which covers the end of the insertion body (8, 9) pointing away from the body (2).

12. Container according to Claim 11, characterised in that the handle part (13) projects in the longitudinal direction of the head (3) away from the latter.

## Revendications

1. Procédé de soufflage destiné à la fabrication, dans des conditions stériles, d'un récipient (1) fermé contenant une substance et formé d'un corps (2) prolongé par une tête (3), dont une partie est détachable pour l'ouverture du récipient (1), dans lequel procédé, développé pour réaliser un récipient formé d'un corps (2) avec un diamètre sensiblement supérieur au diamètre de la tête (3) et d'une zone de jonction entre la tête (3) et le corps (2) qui s'étend au moins pratiquement dans un plan radial,
a) on introduit une section d'un tube extrudé dans un moule pour soufflage muni d'un fond (19) mobile dans le sens axial, le diamètre du tube étant à l'origine inférieur au diamètre de la tête (3),
b) on insuffle de l'air dans le tube après avoir fermé l'extrémité du tube formant le fond du récipient (1), et, en même temps, on déplace le fond du moule (19) contre la coquille moulant la tête (18),
c) on moule la tête (3), qui sera fermée de manière étanche en l'appuyant contre la surface latérale d'une pièce encastrée (8, 9), éventuellement insérée,
caractérisé en ce que
d) après le remplissage du récipient (1) et l'insertion de la pièce à encastrer (8, 9), éventuellement dans la section du tube destinée à former la tête (3), le fond du moule (19) est déplacé encore davantage vers la coquille moulant la tête (18), jusqu'à ce que le récipient (1) ne contienne plus d'air et
e) le fond du moule (19) est déplacé par un premier mouvement jusqu'à la position correspondant à la taille du récipient (1) à l'état fermé et, pendant le processus de remplissage, ledit fond du moule exécute un mouvement de retour sur une distance donnée.

2. Procédé selon la revendication 1, caractérisé en ce que, après le remplissage, le fond du moule (19) est déplacé vers la tête (3) sur la distance parcourue pendant le mouvement de retour.

3. Procédé selon la revendication 1 ou 2, caractérisé en ce que, au cours du premier déplacement du fond du moule (19), l'air est d'abord insufflé avec une pression réduite et, juste avant que ledit fond du moule atteigne sa position finale, la pression est augmentée jusqu'à atteindre la valeur de la pression atmosphérique.

4. Procédé selon l'une quelconque des revendications 1 à 3, caractérisé en ce que, après le remplissage du récipient (1) et pendant le déplacement consécutif du fond du moule (19) vers la tête (3), l'air et l'éventuel excédent de la substance introduite sont de préférence aspirés.

5. Procédé selon la revendication 4, caractérisé en ce que le processus d'aspiration de l'air et de l'excédent de la substance introduite est exécuté par l'intermédiaire de la pièce encastrée (8, 9), le cas échéant.

6. Procédé selon l'une quelconque des revendications 1 à 5, caractérisé en ce que la pièce encastrée (8, 9), éventuellement insérée, est soudée le long de sa périphérie contre la tête (3) qui l'enveloppe.

7. Récipient réalisé selon le procédé conforme à l'une quelconque des revendications 1 à 6, caractérisé en ce que l'épaisseur de la paroi dans la zone qui forme la surface latérale (2') du corps (2) est sensiblement inférieure à l'épaisseur de la paroi dans la zone de jonction (5) entre le corps (2) et la tête (3).

8. Récipient selon la revendication 7, caractérisé en ce que les deux zones formant le fond (4) et la zone de jonction (5) entre le corps (2) et la tête (3) sont conçues de telle sorte que l'une est munie au moins d'une cavité (7) et l'autre au moins d'un amas de matière (6) formant une saillie orientée vers ladite cavité.

9. Récipient selon la revendication 7 ou 8, caractérisé en ce que la pièce encastrée (8, 9) est divisée ou peut être divisée en deux parties dans le sens transversal et cette ligne de partage se situe dans la zone d'un point destiné à la rupture de la tête.

10. Récipient selon la revendication 9, caractérisé en ce qu'au moins la face extérieure de la partie (8) de la pièce encastrée (8, 9), laquelle partie reste attachée à la tête (3) au moment de l'ouverture du récipient (1), est munie d'au moins une rainure annulaire et/ou un bourrelet annulaire (11), afin de réaliser un assemblage par engagement positif dans le sens axial entre la pièce encastrée (8, 9) et la tête (3).

11. Récipient selon l'une quelconque des revendications 7 à 10, caractérisé en ce qu'une surface de prise (13) a été moulée contre la partie (3') de la tête (3) qui recouvre l'extrémité de la pièce encastrée (8, 9), opposée au corps (2).

12. Récipient selon la revendication 11, caractérisé en ce que la surface de prise (13) forme une saillie dans le sens longitudinal de la tête, au-dessus de cette dernière.
